(19) **Europäisches Patentamt European Patent Office Office européen des brevets**

(11) **EP 1 978 980 B1**

(12) **EUROPEAN PATENT SPECIFICATION**

(45) Date of publication and mention
of the grant of the patent:
**09.04.2014 Bulletin 2014/15**

(51) Int Cl.:
*A61K 36/185* *(2006.01)*     *A61K 36/28* *(2006.01)*
*A61K 36/738* *(2006.01)*     *A61P 31/18* *(2006.01)*
*A61P 37/04* *(2006.01)*

(21) Application number: **06706513.6**

(22) Date of filing: **31.01.2006**

(86) International application number:
**PCT/EP2006/000820**

(87) International publication number:
**WO 2007/087825 (09.08.2007 Gazette 2007/32)**

(54) **USE OF A COMBINATION OF ROSA SP., URTICA DIOICA AND TANACETUM VULGARE EXTRACTS, FURTHER COMPRISING SELENIUM AND UREA AND HAVING BEEN EXPOSED TO A PULSED ELECTROMAGNETIC FIELD, FOR THE PREPARATION OF A MEDICAMENT FOR TREATMENT OF HIV INFECTIONS**

VERWENDUNG EINER KOMBINATION AUS ROSA SP., URTICA DIOICA UND TANACETUM VULGARE EXTRAKTEN, DIE  SELEN UND HARNSTOFF ENTHALTEN UND EINEM GEPULSTEN ELEKTROMAGNETISCHEN FELD AUSGESETZT WORDEN SIND, ZUR HERSTELLUNG EINES MEDIKAMENTS ZUR BEHANDLUNG VON HIV-INFEKTIONEN

UTILISATION D'UNE COMBINAISON D'EXTRAITS DE ROSA SP., URTICA DIOICA ET TANACETUM VULGARE, COMPRENANT DU SELENIUM ET DE L'UREE ET AYANT ÉTÉ EXPOSÉS A UN CHAMP ÉLECTROMAGNÉTIQUE PULSÉ, POUR LA PREPARATION D'UN MEDICAMENT POUR LE TRAITEMENT D'INFECTIONS À VIH

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**15.10.2008 Bulletin 2008/42**

(73) Proprietor: **Parsroos Co.**
**Shahrak-e-Gharb, Tehran 14678-76744 (IR)**

(72) Inventors:
• **NOVITSKY, Yury Alexevich**
**Ryazan Province (RU)**
• **MADANI, Hessamedin**
**Tehran (IR)**
• **GHARIBDOUST, Farhad**
**Tehran (IR)**
• **FARHADI, Mohammad**
**Tehran (IR)**
• **FARZAMFAR, Bardia**
**Tehran (IR)**
• **MOHRAZ, Minoo**
**Tehran (IR)**

• **SADEGHI, Dr. Behnam**
**Tehran (IR)**

(74) Representative: **Scholz, Volker et al**
**Boehmert & Boehmert**
**Pettenkoferstrasse 20-22**
**80336 München (DE)**

(56) References cited:
• **DATABASE WPI Week 200303 Derwent Publications Ltd., London, GB; AN 2003-037812 XP002411987 & RU 2 191 824 C2 (ILINA, A.E. ET AL.) 27 October 2002 (2002-10-27)**
• **DATABASE WPI Week 200153 Derwent Publications Ltd., London, GB; AN 2001-487702 XP002411988 & RU 2 171 284 C2 (KIROV, E.I.) 27 July 2001 (2001-07-27)**
• **DATABASE WPI Week 198607 Derwent Publications Ltd., London, GB; AN 1986-047590 XP002411989 & SU 1 169 657 A (AS SIBE BURYATSK; MEDICINAL PLANTS RES; PHARMACE RES INST) 30 July 1985 (1985-07-30)**

**(Cont. next page)**

- DATABASE WPI Week 200118 Derwent Publications Ltd., London, GB; AN 2001-180438 XP002411990 & RU 2 160 596 C1 (FITO-EM AGRO-IND FIRM STOCK CO) 20 December 2000 (2000-12-20)

**Description**

The present invention refers to a method for preparing a herbal extract from *Rosa sp., Urtica dioica and Tanacetum vulgare,* comprising a treatment by pulsed electromagnetic field. The herbal extract is further characterized by added selenium and urea. The present invention further refers to a use of herbal extracts, prepared by said method HIV infection and AIDS.

**Background of the invention**

The acquired immunodeficiency syndrome (AIDS) is a chronic life threatening disease caused by the human immunodeficiency virus (HIV). This retrovirus can be further specified, wherein HIV-1 is the cause of AIDS in the Western hemisphere and in Europe, while HIV-2 is the major cause of AIDS in Africa and Southeast Asia.

In more detail, AIDS is characterised by a series of symptoms becoming evident at later stages of the HIV infection. Without any treatment, the incubation period, i.e. the time period between HIV infection and clinical manifestation of AIDS, is about 10 years. An HIV infection causes progressive impairment of the immune system, finally resulting in immunodeficiency. Accordingly, the most important clinical symptoms of AIDS are opportunistic infections and, furthermore, characteristic malignancies such as Kaposi's sarcoma, HIV encephalopathy and HIV-associated wasting syndrome. The immunodeficiency is based on the loss of the CD4$^+$ T-cells that are essential for both cell-mediated and antibody-mediated immunity. The quantitative analysis of CD4$^+$ T-lymphocytes in the circulating blood has been the crucial method for detecting and evaluating HIV infection and AIDS since the beginning of the epidemic. For determination of the severity of the disease, prognosis and therapeutic observation, the percentage of CD4$^+$ T-lymphocytes and their changes over time (slope) serve as valuable parameters.

First cases of AIDS were reported in the early eighties of the last century. Meanwhile, AIDS has spread worldwide and, moreover, is epidemic in some regions, particularly in some of the developing countries. Today, about 40 million persons are infected by HIV with approximately 2.2 million children among them. In 2004, about five million new infections were noted, and about 3.1 million persons died from AIDS, 510 000 of them were children (according to the World Health Organization WHO).

Since 1995, the number of medicaments for the treatment of AIDS has trebled. Nowadays, combination therapies are of common use providing increased efficiency and deceased side-effects. The development of new active agents or drugs and therapeutic concepts were successful in slowing the progression of the disease, reversing the symptoms of the late stages of the disease and preventing the infection of babies born to infected mothers. The so-called "highly active anti-retroviral therapy" (HAART), a combined therapy comprising three or more drugs, has become a standard therapy. It was shown that HAART delays the progression of AIDS and reduce mortality. Usually, two of the drugs involved in HAART target the reverse transcriptase and one drug targets the viral protease. Well established reverse transcriptase inhibitors are nucleoside analogues such as zidovudine (AZT, Retrovir®), lamivudine (Epivir®, and didanosine (Videx®). Such transcriptase inhibitors are incorporated into the growing DNA strand which the consequence that further DNA synthesis is prevented. Other reverse transcriptase inhibitors are known, e.g., Viramune®, that inhibit the enzyme by other mechanisms. Protease inhibitors block the viral protease so that the proteins needed for assembly of new viruses cannot be cleaved from the large protein precursor. Examples of these kind of drugs are indinavir (Crixivan®, saquinavir (Invirase®, ritonavir (Norvir®), and nelfinavir mesylate (Viracept®). Further drugs useful for therapeutic intervention are fusion inhibitors, e.g. enfuvirtide (Fuzeon®) and integrase inhibitors.

Despite the great advances in the therapy of HIV infection and AIDS, there are still several disadvantages and drawbacks. The drugs currently used are very expensive and thus, they do not only drain resources in affluent countries, but are simply unavailable in the many poor countries where the epidemic rages. They have many unpleasant side-effects (e.g. nausea, diarrhea), however, they also may exert severe side-effects (e.g. liver and pancreas damage, sometimes with fatal outcome). They demand a very complicated dosing regimen, e.g. over a dozen pills a day (not counting those needed to cope with the accompanying opportunistic infections). Finally, they often lose effectiveness as they select for the emergence of drug-resistant virions in the patient.

Consequently, there is a need in alternative, improved or superior pharmaceutical means providing an amelioration of the AIDS symptoms, a retardation of AIDS manifestation and/or an intervention in the HIV infection. Furthermore, there is a need in more cost efficient pharmaceutical means.

Thus, it is an object of the present invention to provide a pharmaceutically active composition useful in the treatment of viral infections and associated symptoms and conditions, preferably HIV infection and AIDS.

**Summary of the invention**

The object of the present invention is solved by a method for preparing a herbal extract according to claim 1.

RU 2191824 C2 discloses an alcoholic herbal extract of plant material derived from *Rosa carnina fruits, Urtica*

*dioica* leafs and *Tanacetum vulgare* flowers. The herbal extract is to be used as an imunostimulant.

RU 2171284 C2 discloses an ethanolic herbal extract of plant material derived from *Rosa sp.* fruits, flowers or leafs, *Urtica dioica* leafs and *Tanacetum vulgare* leafs, or influences.

SU 1169657 A discloses an ethanolic herbal extract of plant material derived from *Rosa carnina, Urtica dioica* and *Tanacetum vulgare.*

The object of the present invention is further solved by a use of the herbal extract according to claim 12.

Preferred embodiments result from the subclaims.

The term "stochastic shape" comprises the meaning that the electromagnetic field pulse is in the form of a noise. Preferably, the electromagnetic field pulse is of rectangular shape and is combined with a sinusoidal wave inside. The "power" (Watt) of the pulsed electromagnetic field means e.g. effective power. The value of the "magnetic field strength" (Tesla) of the pulsed electromagnetic field indicates e.g. from peak to peak.

The effect of "stimulating the immune system" and the conditions of an "impaired immune system" can be determined using methods and parameters known in the art. Targets of such a determination can be any component of the cell-mediated and antibody-mediated immune system such as T-lymphocytes (CD4 and/or CD8 T-lymphocytes), B-lymphocytes, antibodies and components of the complement system. An example of a method for determination is the FACS (fluorescence activated cell sorting) analysis. Preferably, CD4 T-lymphocytes are determined as counts or percentage, most preferably in a time dependent manner.

The term "AIDS", as used herein, refers to a clinical condition having characteristic symptoms associated with later stages of HIV infection.

The term "RNA viral infection", as used herein, refers to an infection by an RNA virus, preferably a retrovirus. Examples for RNA viruses considered by the present invention are be Polio, Coxsackie, Calici, Hepatitis A, Hepatitis C, Hepatitis D, Hepatitis E, Entero, Rhino, Rubella, CEE (central European encephalitis), Influenza, RS (respiratory syncitial), Parainfluenza, Measle, Mumps, Corona, Arena, Lassa, Bunya, Hanta, Rhabdo, Filo, Borna, HTLV (human T-cell leukaemia), and Rota virus.

The term "pharmaceutical composition", as used herein, is intended to comprise the herbal extract. Also considered is a pharmaceutical composition comprising at least one pharmaceutically active component of the herbal extract and/or at least one derivative or analogon of said active component and corresponding salts thereof.

The pharmaceutical composition can be, for example, in a liquid form, e.g. a solution, syrup, elixier, emulsion and suspension, or in a solid form, e.g. a capsule, caplet, tablet, pill, powder, and suppository. Granules or semi-solid forms and gelcaps are also considered. In case that the pharmaceutical composition is a liquid or a powder, the dosage unit optionally is to be measured, e.g. in the dosage unit of a teaspoonful. In addition to the herbal extract or the pharmaceutically active component, the pharmaceutical composition can comprise, for example, flavouring agents, sweeteners, dyes, preservatives, stabilizers, colouring agents, diluents, suspending agents, granulating agents, lubricants, binders and disintegrating agents. A tablet, for example, can be coated. A liquid to be injected should be sterile. Also considered are transdermal delivery systems and liposomal systems. All of the formulations mentioned can be intended for immediate release, timed release and sustained release.

The term "pharmaceutically acceptable", as used herein, means at least non-toxic. The "pharmaceutically acceptable carrier", as meant in the present disclosure, may take a wide variety of forms depending upon the desired route of administration. The term comprises conventional pharmaceutical diluents such as water or ethanol and conventional tableting ingredients such as com starch, lactose, sucrose, sorbitol, talc, stearic acid, magnesium stearate, dicalcium phosphate or gums.

Administration of the pharmaceutical composition can use different routes, such as oral, sublingual, parenteral, intravenous, intraperitoneal, nasal, vaginal, rectal, subcutaneous, intradermal, intramuscular and topic. A dosage unit can be administered once or several times a day, week or month. The delivery can also be continuously by infusion or through a transdermal sustained release system, for example.

Thus, herein provided is a combinatory herbal extract from *Rosa sp., Urtica dioica* and/or *Tanacetum vulgare* treated by electromagnetic field radiation and further comprising added selenium and urea. Clinical data showed a beneficial effect of the extract in the treatment of AIDS. Studies conducted both in *vitro* and in experimental animal models revealed that the extract does not exert toxicity, mutagenicity, or oncogenicity. Pregnancy, however, is a contraindication.

**Detailed description of the invention**

The invention will now be described in more detail by the following examples with the intention to exemplify the invention. The examples, however, are not intended to have any limiting effect on the subject-matter of the claims or on the scope of protection.

EXAMPLE 1: Preparation of raw herbal extracts

Leaves and small stems of nettle (*Urtica dioica*) and tansy (*Tanacetum vulgare*) are collected from wild fields. After separation of useful parts and initial cleaning, the material is dried on a wooden network in a dark place for 3-4 days, preferably at 42°C. In dried condition, the plant material should be green without any change in colour, and leaves and stems should be brittle. For extraction, airtight glass vessels are used. The dried plant material is broken into small pieces (2-5 cm) and placed into the glass vessels such that there is no space left. After packing (compressing), EtOH (96 %; herein, % of an ethanolic solution refers to % by volume)) is added until the vessel is filled completely. The vessels are placed into an incubator (37-45°C, preferably 42°C) for 20-40 days until a dark green solution appears.

For the extraction of wild rose (*Rosa canina*), dried fruits are used. Other species of *Rosa sp.* can be used alternatively or in addtion. The fruits are filled into air-tight vessels up to a half and EtOH is added. The vessels are kept in an incubator (37-45°C, preferably 42°C) for 20-40 days until an orange-red coloured extract appears.

After the incubation period, when the plant material is colourless, the extracts are collected by separating them from plant material using a cloth filter.

EXAMPLE 2: Electromagnetic treatment

The extract of *Rosa canina* is exposed to an electromagnetic field for 3 min. Then, 50-70 ml of the radiated *Rosa canina* extract is transferred to 2 l of *Urtica dioica* and *Tanacetum vulgare* extracts, respectively. To each litre of the combined *Urtica/Rosa* and *Tanacetum/Rosa* extracts, respectively, 16 mg of selenium and 150 mg urea is added. Then, the vessels are sealed again and kept in the incubator for 24 h at 42°C. After incubation, the vessels are exposed 4 times to an electromagnetic field, 3 min each, and are pooled together. The resulting extract is passed sequentially through a 5, 0.45 and 0.22 $\mu$m filter, respectively, and partitioned to sterile vials. After labelling and packaging, the herbals extract is ready for use.

The electromagnetic field, to which the raw extracts are exposed, is pulsed, powerful and monopolar in that the direction of the electric current generated in a Magnetic Impulse Generator (MIG) apparatus doesn't change. The pulsed magnetic field has a very high frequency ranging from 5 kHz-750 kHz. In this example, the pulse of a rectangular shape used. Nevertheless, a sinusoidal or stochastic shape may beconsidered as well. Preferably, the pulse is of rectangular shape and is combined with a sinusoidal wave inside. Although it is not intended to be bound to any theory, it is hypothesised that the special kind of the produced pulse causes some changes in the physical configuration of atoms in the molecules and/or arrangements of molecules thus leading to altered chemical properties.

In the preparation of the herbal extracts, 3-4 times radiation of electromagnetic pulses of high frequency is used for 2-5 min each. The electrical power (e.g. effective power) of the pulses is about 20 to 100 Watt, and the best effect is obtained at 45 Watt.

EXAMPLE 3: Pre-clinical studies

In pre-clinical studies, the herbal extract was studied for acute toxicity and chronic toxicity. For studying the drug acute toxicity, BALB/c mice and Wistar rats were injected with a single intramuscular (i.m.) injection. The drug chronic toxicity was studied during 3 months in Wistar rats and during 1 month in dogs. The potential mutagenic, embryotoxic, teratogenic, allergenic and immunotoxic properties of the herbal extract as well as its effect on the reproductive function were investigated.

As a result of the conducted experiments, it was evident that the herbal extract is a low-toxic drug following a single i.m. injection to BALB/c mice and Wistar rats. Under acute toxicity experimental conditions, the $LD_{50}$ of the extract administered intraperitoneally (i.p.) as an 1:5 dilution in normal saline was 51-54 ml/kg in rats and 56-59 ml/kg in mice.

When BALB/c mice or Wistar rats were injected i.m. or i.p. with the herbal extract, no specific or sexual differences were observed in the sensitivity of the test animals to the toxic effects of the extract. The pattern of intoxication of BALB/c mice and Wistar rats in doses equal to $LD_{50}$ was similar to the pattern of ethyl alcohol poisoning; ethanol was used as a solvent at high concentrations in the herbal extract preparation. Studying the chronic toxicity of the herbal extract following its application i.m. to rats in doses of 0.07 and 0.21 ml/kg once a day for 3 months (10 and 30 times higher than the daily dose for humans) and to dogs in a dose of 0.07 ml/kg (10 times higher than the daily dose for humans) once a day for 1. month, no damaging effect was observed in the basic organs and systems of the animal body.

The herbal extract obviously does not possess mutagenicity and it doesn't affect the reproductive function of animals. When the herbal extract was applied i.m. once a day to rats during pregnancy (from the first day of pregnancy up to birth) in doses of 0.21 ml/kg, the drug exhibited embryotoxic and teratogenic properties. Therefore, it is contraindicated in pregnancy.

Furthermore, using doses of 0.07 and 0.14 ml/kg administered to guinea pigs every other day within 10 days, the herbal extract did not exhibit allergenic or immunotoxic properties.

In conclusion, the results of the toxicological experiments indicate that the herbal extract can be used for systematic clinical trials with considering its contraindication in pregnancy.

3.1. Acute toxicity

The studies were performed with 128 BALB/c mice (males and females, body weight 18-20 g) and 47 Wistar rats (males and females, body weight 180-220 g) using i.m. injection of the herbal extract in mice and i.p. injection in mice and rats. The herbal extract was diluted 1:10 and 1:5 in sterile normal saline, and then different volumes of these dilutions were applied to the test animals. The experimental animals were then observed for 14 days to determine possible toxic effects of the preparation.

The toxicity of the herbal extract following a single injection to the test animals were determined using a double-stage method: first, an approximate $LD_{50}$ was established by the Deihman and Leblanc technique followed by the determination of the precise indices of $LD_{16}$, $LD_{50} \pm SD$ and $LD_{84}$ by probit-analysis according to Litchfield and Wilcoxon.

The conducted experiments revealed that a single i.m. injection of the herbal extract diluted 1:10 in normal saline in doses of 25-50 ml/kg to BALB/c mice doesn't cause intoxication and death of animals. When the doses increased to 75-100 ml/kg, a decrease in motor activity was observed, but no animal death. Injection of the herbal extract diluted 1:5 in normal saline in doses of 25-50 ml/kg was followed by pain, profound depression and finally animal death after few hours. The intoxication profile of the herbal extract in mice was similar to that of their poisoning by ethyl alcohol (37.3 ml/kg) which is present at high concentrations in the herbal extract as a vehicle. The results in Wistar rats were similar to those observed in BALB/c mice.

Table 1. Parameters of acute toxicity of the herbal extract and ethanol (96%) diluted 1:5 in normal saline at different concentrations following i.m. or i.p. administration to experimental animals.

| Route of administration | Toxicity indices [ml/kg] | | | | | |
|---|---|---|---|---|---|---|
| | $LD_{16}$ | $LD_{50} \pm SD$ | $LD_{84}$ | $LD_{16}$ | $LD_{50} \pm SD$ | $LD_{84}$ |
| | Males | | | Females | | |
| BALB/c mice, herbal extract | | | | | | |
| i.m. | 53 | 66±4.6 | 82 | 49 | 62±4.3 | 78 |
| i.p. | 46 | 59±3.8 | 75 | 43 | 56±3.7 | 72 |
| BALB/c mice, ethyl alcohol | | | | | | |
| i.p. | 30.4 | 37.3±2.1 | 44.8 | ND* | ND | ND |
| Wistar rats, herbal extract | | | | | | |
| i.p. | 42 | 54±4.3 | 71 | 38 | 51±4.2 | 67 |
| *ND - not determined. | | | | | | |

The data presented in Table 1 above show e.g. that the toxicity of the herbal extract according to the parameters of toxicometry doesn't differ significantly between i.m. and i.p. injection.

3.2. Chronic toxicity

*3.2.1. Studies in rats*

The toxicity studies on the herbal extract were performed during 3 months by i.m. injection of the preparation to Wistar rats. These experiments were performed with 90 Wistar rats (males and females, body weight 180-200 g), which were divided into 3 groups of 30 animals (15 males and 1.5 females). The first group served as a control (normal saline), the second group received 0.07 ml/kg of the herbal extract, and the third group received 0.21 ml/kg of the herbal extract. The studied doses of the herbal extract were 10 and 30 times higher than the daily therapeutic dose recommended for humans (0.5 ml of the herbal extract diluted in 5 ml of normal saline or 0.007 ml/kg of the preparation).

The results show that i.m. injection of the herbal extract in doses of 0.07 and 0.21 ml/kg has no effect on the general state and behaviour of rats. During the chronic toxicity experiments, statistically significant differences in the amount of erythrocytes, leukocytes, platelets, and hemoglobin levels were not observed in animals receiving 0.07 and 0.21 ml/kg, respectively, of the herbal extract compared to the control (Tables 2 and 3).

Table 2. Hematologic parameters in male rats following 3 months of i.m. injection with the herbal extract.

| Erythrocytes [$\times 10^{12}$/l] | | | |
|---|---|---|---|
| Observation period | Control | Herbal extract | |
| | | 0.07 ml/kg | 0.21 ml/kg |
| Initial value | 7.3±0.6 | 7.4±0.4 | 7.3±0.2 |
| After 1 month | 7.3±0.5 | 7.5±0.4 | 7.7±0.5 |
| After 3 months | 7.6±0.4 | 7.8±0.3 | 7.7±0.6 |
| | | | |
| Leukocytes [$\times 10^{9}$/l] | | | |
| Observation period | Control | Herbal extract | |
| | | 0.07 ml/kg | 0.21 ml/kg |
| Initial value | 11.4±0.7 | 11.0±0.5 | 11.0±0.5 |
| After 1 month | 11.7±0.6 | 11.1±0.3 | 11.2±0.4 |
| After 3 months | 11.2±0.7 | 11.2±0.4 | 11.3±0.6 |
| | | | |
| Platelets [$\times 10^{9}$/l] | | | |
| Observation period | Control | Herbal extract | |
| | | 0.07 ml/kg | 0.21 ml/kg |
| Initial value | 660±30 | 660±30 | 660±30 |
| After 1 month | 672±33 | 663±23 | 669±24 |
| After 3 months | 667±29 | 669±31 | 655±28 |
| | | | |
| Hemoglobin [g/l] | | | |
| Observation period | Control | Herbal extract | |
| | | 0.07 ml/kg | 0.21 ml/kg |
| Initial value | 111±4 | 110±3 | 111±2 |
| After 1 month | 114±3 | 115±4 | 117±5 |
| After 3 months | 116±4 | 115±3 | 118±3 |

Table 3. Hematologic parameters in female rats following 3 months i.m. injection with herbal extract.

| Erythrocytes [$\times 10^{12}$/l] | | | |
|---|---|---|---|
| Observation period | Control | Herbal extract | |
| | | 0.07 ml/kg | 0.21 ml/kg |
| Initial value | 7.2±0.4 | 7.2±0.3 | 7.0±0.3 |
| After 1 month | 7.1±0.4 | 7.4±0.5 | 7.6±0.4 |
| After 3 months | 7.3±0.4 | 7.7±0.4 | 7.8±0.3 |
| | | | |

(continued)

| Leukocytes [$\times 10^9$/l] | | | |
|---|---|---|---|
| Observation period | Control | Herbal extract | |
| | | 0.07 ml/kg | 0.21 ml/kg |
| Initial value | 11.3±0.4 | 11.6±0.5 | 11.4±0.3 |
| After 1 month | 11.5±0.5 | 11.2±0.3 | 11.5±0.5 |
| After 3 months | 11.2±0.6 | 11.1±0.4 | 11.2±0.3 |

| Platelets [$\times 10^9$/l] | | | |
|---|---|---|---|
| Observation period | Control | Herbal extract | |
| | | 0.07 ml/kg | 0.21 ml/kg |
| Initial value | 670±27 | 660±20 | 671±25 |
| After 1 month | 678±24 | 669±27 | 673±22 |
| After 3 months | 667±25 | 668±21 | 662±22 |

| Hemoglobin [g/l] | | | |
|---|---|---|---|
| Observation period | Control | Herbal extract | |
| | | 0.07 ml/kg | 0.21 ml/kg |
| Initial value | 112±4 | 110±3 | 111±4 |
| After 1 month | 114±3 | 118±4 | 119±4 |
| After 3 months | 113±5 | 119±6 | 119±7 |

Under the conditions of chronic toxicity experiments in rats, i.e. i.m. injection of the preparation in doses of 0.07 ml/kg and 0.21 ml/kg, no significant changes in total protein blood serum level were observed. The absence of damaging effects by prolonged treatment with the herbal extract in doses of 0.07 and 0.21 ml/kg to rats is supported by the stable activity of hepatic enzymes, such as aspartate and alanine aminotransferases, lactate dehydrogenase, and alkaline phosphatase during 3 months of the chronic toxicity experiments. Prolonged administration of the herbal extract to rats didn't influence the levels of bilirubin, cholesterol, triglycerides, urea, creatinine, and glucose in the blood serum of the experimental animals.

Prolong injection of the preparation in doses of 0.07 and 0.21 ml/kg, diluted 1:10 in normal saline, into the thigh muscles of the animals didn't have locally irritating effects.

Macroscopic examinations did not show any toxic or toxico-allergic effects of the herbal extract on this group of animals.

No pathological changes of the internal organs (brain, pituitary gland, thymus, trachea, lungs, heart, esophagus, spleen, liver, pancreas, adrenal glands, kidney, stomach, and reproduction organs in female and male) were observed in this group of animals during 3 months after i.m. injection of the herbal extract in a dose of 0.21 ml/kg.

*3.2.2. Studies in dogs*

The experiments were conducted in 8 dogs (males, initial body weight 12-14.5 kg) which were divided into 2 groups, 4 animals in each: first group - control, second group - herbal extract 0.07 ml/kg. The applied dose of the preparation was 10 times higher than the highest daily therapeutic dose for humans. The studied preparation in a dose of 0.07 ml/kg was diluted in sterile normal saline 1:10, and then it was injected into thigh muscles of dogs once a day for 1 month.

Electrocardiogram (ECG) examination performed before the beginning of the experiments in dogs and 1 month after the beginning of the treatment using a dose of 0.07 ml/kg did not reveal an increase in the heart rate or changes in the ECG parameters. All the ECG parameters didn't change before and after experiments in treated compared to control animals (Table 4):

Table 4. ECG parameters of the dogs receiving the herbal extract by i.m. injection in a dose of 0.07 ml/kg during 1 month.

| ECG Parameters | Initial value | | After 1 month | |
|---|---|---|---|---|
| | Control | Herbal extract 0.07 ml/kg | Control | Herbal extract 0.07 ml/kg |
| R-R, m sec | 467±38 | 458±35 | 466±32 | 462±22 |
| P-Q, m sec | 86±12 | 89±16 | 85±21 | 89±16 |
| Q-T, m sec | 139±19 | 146±18 | 138±15 | 139±15 |
| QRS, m sec | 40±6 | 39±3 | 39±4 | 39±6 |
| ST, m sec | 0.4±0.2 | 0.4±0.2 | 0.4±0.2 | 0.4±0.2 |
| TP, m sec | 189±19 | 1392±28 | 186±22 | 194±23 |
| P, m sec | 0.30±0.03 | 0.29±0.05 | 0.26±0.04 | 0.28±0.04 |
| R, m sec | 1.38±0.12 | 1.39±0.24 | 1.42±0.22 | 1.38±0.23 |
| T, m sec | 0.30±0.06 | 0.32±0.06 | 0.29±0.06 | 0.28±0.05 |
| Heart rate/min | 139±15 | 145±15 | 138±12 | 146±12 |

The results showed that administration of the herbal extract to dogs by i.m. injection every day during 1 month in a dose of 0.07 ml/kg (10 times higher than a daily dose for humans) doesn't have any effect on the general state and behaviour of animals, and this treatment also doesn't change the functional state of the main organs and systems of the animal body.

According to our data of histological examinations no toxic or local irritating effects of the herbal extract were revealed during 1 month of i.m. injection in a dose of 0.07 ml/kg in dogs.

3.3. Mutagenicity

3.3.1. Gene mutations in microorganisms (Ames test)

Evaluation of the mutagenic activity of the herbal extract was carried out by means of the technique of the ability of a substance to induce gene mutations in indicator microorganisms in the system of metabolic activation in *vitro* and without such system. Plate method of identification of mutations was used. This method was provided by Ames et al., and we used three autotrophic stains on histidine, namely *Salmonella typhimurium* TA 98, TA 100 and TA 1537, as indicator microorganisms.

In order to conduct the Ames test, dilutions of the herbal extracts were obtained in the following way: the initial solution of the preparation was measured and weighed and diluted with distilled water to a concentration of 10 mg/ml. Further dilutions were prepared in distilled water and added to Petri dishes. The concentrations of the preparation from 0.1 to 1000 µg/dish were examined. Distilled water was used as negative control.

Table 5. The effect of the herbal extract on the bacterial indicator strain TA 98 in the Ames test.

| Studied substance | Dose µg/dish | TA 98 strain | | | | | | | |
|---|---|---|---|---|---|---|---|---|---|
| | | -S9 | | | | +S9 | | | |
| | | $M_{i1,2,3}$ | Mean | $M_O/M_K$ | MA | $M_{i1,2,3}$ | Mean | $M_O/M_K$ | MA |
| Negative control (H$_2$O) | 0 | 33 29 37 | 32.8 | 1 | - | 403631 | 35.5 | 1 | - |
| Positive control (2AA) | 20 | 0 0 0 | 0 0 0 | 0 0 0 | - | 1211 1280 1140 | 1209 | 34.0 | + |
| Positive control (ANQO) | 0.5 | 568 567 614 | 6 | 17.8 | + | 0 0 0 | 0 0 0 | 0 0 0 | - |

(continued)

| Studied substance | Dose μg/dish | TA 98 strain | | | | | | | |
|---|---|---|---|---|---|---|---|---|---|
| | | -S9 | | | | +S9 | | | |
| | | $M_{i1,2,3}$ | Mean | $M_O/M_K$ | MA | $M_{i1,2,3}$ | Mean | $M_O/M_K$ | MA |
| Herbal extract | 0.1 | 35 32 35 | 34.0 | 1.04 | - | 30 42 45 | 38.4 | 1.08 | - |
| | 1.0 | 29 32 37 | 32.5 | 0.99 | - | 40 35 29 | 34.4 | 0.97 | - |
| | 10.0 | 28 34 38 | 33.1 | 1.01 | - | 36 35 32 | 34.3 | 0.97 | - |
| | 100.0 | 27 30 26 | 27.6 | 0.84 | - | 19 27 31 | 25.1 | 0.71 | - |
| | 1000.0 | 29 28 33 | 29.9 | 0.91 | - | 30 28 39 | 32 | 0.90 | - |

Conventional signs and abbreviations: $M_i$ - the number of revertants per dish, M - average geometrical number, $M_0/M_k$ - the ratio of the number of revertants in the test and the number of revertants in negative control, MA mutagenic activity: "+" - the presence of activity, "-"-the absence of activity.

Table 6. The effect of the herbal extract on the bacterial indicator strain TA 100 in the Ames test.

| Studied substance | Dose μg/dish | TA 100 strain | | | | | | | |
|---|---|---|---|---|---|---|---|---|---|
| | | -S9 | | | | +S9 | | | |
| | | $M_{i1,2,3}$ | Mean | $M_O/M_K$ | MA | $M_{i1,2,3}$ | Mean | $M_O/M_K$ | MA |
| Negative control ($H_2O$) | 0 | 144 223 228 | 194.2 | 1 | - | 188 287 206 | 223.2 | 1 | - |
| Positive control (2AA) | 20 | 0 0 0 | 0 0 0 | 0 0 0 | - | 2712 2008 2200 | 2288.2 | 10.2 | + |
| Positive control (Sodium azide) | 2.0 | 1240 1368 1288 | 1297.6 | 6.68 | + | 0 0 0 | 0 0 0 | 0 0 0 | - |
| Herbal extract | 0.1 | 185 154 225 | 185.8 | 0.96 | - | 190 253 284 | 239.0 | 1.07 | - |
| | 1.0 | 154 169 213 | 177 | 0.91 | - | 176 185 222 | 193.3 | 0.87 | - |
| | 10.0 | 153 201 242 | 195.2 | 1.00 | - | 175 185 193 | 184.2 | 0.82 | - |
| | 100.0 | 165 221 213 | 198.0 | 1.02 | - | 146 202 201 | 181.0 | 0.81 | - |
| | 1000.0 | 209 207 202 | 206 | 1.06 | - | 131 136 194 | 151.2 | 0.68 | - |

Table 7. The effect of the herbal extract on the bacterial indicator strain TA 1537 in the Ames test.

| Studied substance | Dose $\mu$g/dish | TA 1537 strain | | | | | | | |
|---|---|---|---|---|---|---|---|---|---|
| | | -S9 | | | | +S9 | | | |
| | | $M_{i1, 2, 3}$ | Mean | $M_O/M_K$ | MA | $M_{i1, 2, 3}$ | Mean | $M_O/M_K$ | MA |
| Negative control (H$_2$O) | 0 | 6 6 10 | 7.11 | 1 | - | 9 9 11 | 9.62 | 1 | - |
| Positive control (2AA) | 20 | 0 0 0 | 0 0 0 | 0 0 0 | - | 126 144 118 | 128.9 | 13.4 | + |
| Positive control (9AA) | 20 | 5448 5632 5752 | 5609.3 | 788.5 | + | 0 0 0 | 0 0 0 | 0 0 0 | - |
| Herbal extract | 0.1 | 6 6 4 | 5.24 | 0.74 | - | 7 6 8 | 6.95 | 0.72 | - |
| | 1.0 | 8 7 7 | 7.32 | 1.03 | - | 10 8 8 | 8.62 | 0.90 | - |
| | 10.0 | 10 10 9 | 9.65 | 1.36 | - | 5 3 3 | 3.56 | 0.37 | - |
| | 100.0 | 5 5 5 | 5.00 | 0.70 | - | 8 7 9 | 7.96 | 0.83 | - |
| | 1000.0 | 10 8 9 | 8.96 | 1.26 | - | 7 6 5 | 5.94 | 0.62 | - |

From the data given above we can conclude that the herbal extract in concentrations of 0.1-1000 $\mu$g/dish does not cause an increase in the number of revertants in *Salmonella typhimurium* strains TA 98, TA 100, TA 1537. Thus, the herbal extract does not have a mutagenic effect according to the Ames test.

*3.3.2. Dominant lethal mutations in murine germ cells*

The experiments were carried out in order to evaluate the potential mutagenic properties of the herbal extract in the experiments aimed at studying dominant lethal mutations in mice hybrids $F_1$ (CBA x $C_{57}Bl_6$).

The herbal extract diluted in normal saline was administered i.m. to male mice in a dose of 0.7 ml/kg. This dose was 100 times higher than the recommended daily dose for humans (0.007 ml/kg).

Table 8. The results of the ability of the herbal extract to induce dominant lethal mutations in mice germ cells.

| Stages of Spermatogenesis | Dose | Total No.of females | No. of pregnant females | Fertility (%) | Post implantation losses | $\chi^2$ |
|---|---|---|---|---|---|---|
| Normal spermatozoids | 0 (control) | 41 | 33 | 80.5 | 0.112 | |
| | Herbal extract 700 $\mu$l/kg | 39 | 34 | 75.6 | 0.011 | |
| Late spermatids | 0 (control) | 42 | 30. | 71.4 | 0.082 | |
| | Herbal extract 700 $\mu$l/kg | 39 | 29 | 74.4 | 0.037 | |
| Early spermatids | 0 (control) | 42 | 38 | 90.5 | 0.055 | |
| | Herbal extract 700 $\mu$l/kg | 38 | 29 | 76.3 | 0.143 | 9.87 |
| Repeated experiments | Herbal extract 700 $\mu$l/kg | 32 | 24 | 75.0 | 0.061 | 0.018 |
| Average of 2 experiments | Herbal extract 700 $\mu$l/kg | 35 | 27 | 90.5 | 0.099 | 2.93 |

(continued)

| Stages of Spermatogenesis | Dose | Total No.of females | No. of pregnant females | Fertility (%) | Post implantation losses | $\chi^2$ |
|---|---|---|---|---|---|---|
| Sum of the experiments and repeated experiments | Herbal extract 700 $\mu$l/kg | 70 | 53 | 76.0 | 0.097 | |
| Results of the 4th week | Herbal extract 700 $\mu$l/kg | 33 | 33 | 100 | 0.040 | |

As can be seen from Table 8, the level of post implantation losses in animals undergoing the effect of a single i.m. injection of the herbal extract in dose of 700 $\mu$l/kg doesn't exceed the level in control animals.

*3.3.3. Chromosomal aberrations in murine bone marrow cells*

The essence of this method consists in an evaluation of the effect of the examined substance introduced into the body of an animal on the genetic system of bone marrow cells sensitive to effects of chemical agents and physical factors. Chromosomal aberrations were analyzed after the administration of the herbal extract according to the scheme disclosed in "Instructions for experimental (pre-clinical) study of new pharmacologic substances".

Table 9. Structural disturbances of chromosomes in the bone marrow cells of mice under the effect of the herbal extract.

| Variants | No. | Cell number | | Aberrations | | | | |
|---|---|---|---|---|---|---|---|---|
| | | Counted | With aberrations | Fragments | | Exchange | Numbers | Gaps |
| | | | | Single | Pair | | | |
| Herbal extract, 700 $\mu$l/kg (after 24 hours counted) | 1 | 100 | 0 | 0 | 0 | 0 | 0 | 0 |
| | 2 | 100 | 1 | 0 | 0 | 0 | 0 | 1 |
| | 3 | 100 | 2 | 2 | 0 | 0 | 0 | 0 |
| | 4 | 100 | 2 | 2 | 0 | 0 | 0 | 0 |
| | 5 | 100 | 0 | 0 | 0 | 0 | 0 | 0 |
| Total | | 500 | 5 | 4 | 0 | 0 | 0 | 1 |
| Herbal extract, 700 pvkg 4 days injection (6 hours after 4th day counted) | 1 | 100 | 0 | 0 | 0 | 0 | 0 | 0 |
| | 2 | 100 | 1 | 0 | 0 | 0 | 0 | 1 |
| | 3 | 100 | 2 | 2 | 0 | 0 | 0 | 0 |
| | 4 | 100 | 1 | 1 | 0 | 0 | 0 | 0 |
| | 5 | 100 | 0 | 0 | 0 | 0 | 0 | 0 |
| Total | | 500 | 4 | 3 | 0 | 0 | 0 | 1 |
| Normal saline | 1 | 100 | 2 | 2 | 0 | 0 | 0 | 0 |
| | 2 | 100 | 1 | 0 | 0 | 0 | 0 | 1 |
| | 3 | 100 | 2 | 1 | 0 | 0 | 0 | 1 |
| | 4 | 100 | 0 | 0 | 0 | 0 | 0 | 0 |
| | 5 | 100 | 0 | 0 | 0 | 0 | 0 | 0 |
| Total | | 500 | 5 | 3 | 0 | 0 | 0 | 2 |

As can be seen from the experimental data, statistically significant differences in the level of chromosomal aberrations were not observed in the bone marrow of mice undergoing the effect of the herbal extract in studied doses as compared to the control. Therefore, according to the test of the chromosomal aberrations in bone marrow cells, the herbal extract doesn't possess mutagenic activity.

*3.3.4. DNA damaging effect in the SOS-chromotest*

One of the tests of DNA damage is the test for determination of the induction of the SOS-response of a bacterial cell to the effect of the agent investigated, the so-called SOS-chromotest. The test is based on the knowledge of SOS-response to DNA damages. The basis of the test is the strain of *E. coli* PQ 37 constructed by means of the association of *LacZ* responsible for the synthesis of the enzyme beta-galactosidase with the gene *sfi*A controlled by the general repressor of the SOS-system. SfiA expression is induced after DNA damage as a part of the SOS-response. In this test, SOS expression is measured according to the qualitative determination of enzyme activity of beta-galactosidase, which can be measured according to colour reaction. The marker of cell growth in this strain is alkaline phosphatase, the activity of which can also be measured according to colour reaction. As a result of analysis, the curves of dependence of the beta-galactosidase synthesis on the concentration of the investigated substance and the curves characterising the changes of bacterial growth in such conditions are obtained. According to these indices, SOS-inducing potency is calculated. This potency reflects the ability of the substance to induce *sfi*A gene expression.

The results obtained showed that the herbal extract did't induce the activation of DNA repair system in any of the investigated concentrations in *E. coli* PQ 37. Thus, the herbal extract doesn't possess a DNA damaging effect.

3.4. Embryotoxicity and teratogenicity

The experiments were conducted in 36 pregnant Wistar rats divided into 2 groups, 18 rats in each: first group - control, second group - herbal extract (in a dose of 0.21 ml/kg that is 30 times higher than the maximal daily dose for humans). The preparation was diluted in normal saline and administered i.m. to rats once a day during all the period of pregnancy (from first day up to birth). The control animals received the appropriate amount of normal saline every day from the first day of pregnancy up to birth. At the 20th day of pregnancy, 70% of pregnant rats were sacrificed by means of dislocation of cervical vertebrate for the subsequent examination of the bony skeletons and internal organs of the fetuses and determination of the indices of pre-implantation and post-implantation death.

Table 10. The changes of body weight of pregnant rats (% of initial).

| Animal groups | 1 week | 2 weeks | 3 weeks |
|---|---|---|---|
| Control | 123 ± 1.9 | 136.7±2.2 | 145.7 ± 2.8 |
| Herbal extract, 0.21 ml/kg | 112.5 ± 1.5* | 120.5 ± 3.4* | 131.6 ± 4.5* |
| Note: * indicates significant difference (P<0.05) | | | |

As summarized in table 10, i.m. injection of the herbal extract to pregnant females in a dose of 0.21 ml/kg didn't influence the increase in the body weight of pregnant females as compared to the control group during all the period of pregnancy.

Such criteria of the evaluation of embryotoxicity of the herbal extract in the duration of pregnancy as the number of alive fetuses, implantation sites, yellow bodies, and embryo body weight were lower in the group of rats receiving the preparation. The index of pre-implantation death in the experimental group was significantly higher than that of the control, but post-implantation death level was lower. Cranio-caudal size of the fetuses of pregnant rats receiving the preparation in dose of 0.21 ml/kg was not statistically different from the appropriate size in the control group (Table 11).

Table 11. The indices of embryotoxic effects of the herbal extract in a dose of 0.21 ml/kg following i.m. administration of the preparation from the first till the 20th day of pregnancy.

| Investigated indices | Control | Herbal extract 0.21 ml/kg |
|---|---|---|
| The duration of pregnancy (days) | 24.5 ± 0.2 | 23.3 ± 0.2* |
| The number of embryos per rat | 9.5 ± 1.3 | 5.5 ± 0.6* |
| The number of yellow bodies per rat | 10.0 ± 1.2 | 6.0 ± 0.8 * |
| Pre-implantation death (%) | 13.5 | 40.8 |
| Post-implantation death (%) | 8 | 5.1 |

(continued)

| Investigated indices | Control | Herbal extract 0.21 ml/kg |
| --- | --- | --- |
| Cranio-caudal size of the fetus (cm) | 3.3 ± 0.1 | 3.0 ± 0.1 |
| The weight of the fetus (g) | 3.0 ± 0.1 | 2.2 ± 0.2 * |
| Note: * indicates significant difference (P<0.05) | | |

Macroscopic and microanatomic examinations (standard incisions according to Wilson-Diban) of fetuses undergoing the effect of the herbal extract in their pre-natal period in a dose of 0.21 ml/kg revealed underdevelopment of the fetus in 6.7% of cases. The frequency of such pathologies as hydronephrosis and hemopericardium in the group of rats receiving the herbal extract during pregnancy exceeded that of the control group.

When analyzing total fetus body preparations stained with alizarin with the purpose of studying the development of bony system in rat fetuses undergoing the effect of the herbal extract in a dose 0.21 ml/kg in the pre-natal period, no developmental defects of the skeleton were revealed. However, inhibition of ossification in the majority of the investigated foci of bone was observed.

In conclusion, it must be noted that i.m. administration of the herbal extract in a dose of 0.21 ml/kg (this dose is 30 times higher than the maximal therapeutic dose for humans) to pregnant rats from the first to the 20th day of pregnancy had a negative effect on the changes of the body weight of pregnant rats and on the duration of pregnancy. Effects of the preparation on indices of embryotoxicity such as the number of live fetuses, embryo weight, the number of implantation sites and yellow bodies, parameters of pre-and post-implantation death, and also the inhibition of ossification of the foci of bone were observed.

During the post-natal development of young rats, significant retardation of physical development was noted. In the experimental group, an underdevelopment of fetuses was revealed in 6.7%. This underdevelopment can be considered as developmental defect of embryos.

Thus, embryotoxic and teratogenic effects of the herbal extract in dose of 0.21 ml/kg were revealed. In connection with this, pregnancy must be considered to be a contraindication to the administration of the preparation.

3.5. Effect on the reproductive function

Investigations were carried out in Wistar rats (males and females; initial body weight 180-200 g). A group of females, consisting of 60 animals, was divided into two sub-groups: the control group (40 animals) and the experimental group (20 animals). Every day females of the experimental group were injected i.m. with 0.21 ml/kg of the herbal extract during the period of 2 weeks (3-4 of estrous cycle). Before injection, the preparation was diluted in normal saline.

Intramuscular injection, of 0.21 ml/kg of the herbal extract to male and female rats did not change such indices of the reproductive function of rats as amounts of yellow bodies, implantations, live fetuses, and resorptions. The value of pre-implantation and post-implantation deaths was not very different from the control one; it was true both for the females who received the preparation and for the females who were impregnated by the males who had been injected the preparation.

The investigations helped to establish that i.m. injection of 0.21 ml/kg of the herbal extract (that is 30 times higher than the maximal daily dose recommended for humans) does not influence either sexual activity, reproductive indexes (amount of live fetuses, their body weight, amount of yellow bodies, cranio-caudal rate, places of implantation, resorptions), or neonatal development of the rats. Thus, influence of the preparation investigated on the reproductive function of sexually healthy mature rats was not revealed.

3.6. Immunorelated effects

3.6.1. Allergenic and anaphylactic effects

This study was done in accordance with "Methodical instructions for evaluating allergenic properties of pharmacological substances" (Experimental study guide of new pharmacological substances. Moscow, 2000, pp. 25-32).

Investigations were carried out in 15 guinea-pigs (males, body weight is equal to 270-320 g), that were divided into three groups. Each group contained 5 animals: first group - control (normal saline), second group - 0.07 ml/kg of the herbal extract, third group - 0.14 ml/kg of the herbal extract. The preparation of doses used corresponded to 10 and 20 times more than daily doses recommended for a human. The investigations showed that 0.14 ml/kg of the herbal extract (20 times more than the daily therapeutic dose for a human) does not cause an anaphylactic shock upon i.m. injection on the 14th and on the 21st days of sensitization.

*3.6.2. Delayed type hypersensitivity reactions*

Examinations were done on the skin of 15 guinea-pigs, that were of white-coloured skin (males, body weight is equal to 260-310 g). They were divided into 3 groups of 5 animals in each: first group - control, the second group - 0.007 ml/kg of the herbal extract, third group-0.035 ml/kg of the herbal extract. The doses of the herbal extract mentioned above were diluted in sterile normal saline, then mixed with complete Freund's adjuvant in a ratio of 1:1 and then injected into the animals. According to the results of the previous investigations, reaction of hypersensitivity of a delayed-type in the herbal extract doses mentioned was negative for the guinea-pigs.

Forty-nine hybrid mice F1 (CBA*C57B16) (males, body weight is equal to 18-20 g) were divided into 7 groups, each group had 7 animals. The mice were immunized with a subcutaneous (s.c.) injection of RCR (dose: $2 \times 10^8$ cells for a mouse) in an interscapular region. The difference in their mass characterised the degree of edema and intensity of the hypersensitivity reaction of a delayed type. The index of the reaction was calculated according to the formula:

$$U = (P_0 - P_{control} / P_{control}) \times 100$$

where Po is the mass of the experimental foot, and $P_{control}$ is the mass of the control foot.

Data analysis proved that the tested doses of the herbal extract, i.e. 0.18 and 0.07 ml/kg, did not influence the formation of hypersensitivity reaction of delayed type for mice or cellular immunity. Therefore, results of the experiments showed that the herbal extract did not have immunotoxic properties.

*3.6.3. Effects on the mass and number of cells of the popliteal lymph nodes in mice*

To evaluate the allergenic properties of the herbal extract, the method of popliteal lymph nodes weight and mass change for rats was used as a response to an antigenic irritant, a so-called "popliteal lymph node assay", PLNA. Ten hybrid mice F1 (CBA* C57B16) (males, body weight is equal to 18-20 g) were injected with 50 $\mu$l of sterile normal saline (control), and with 0.07 ml/kg of the herbal extract, respectively.

After 7 days weight and cellularity of right and left popliteal lymph nodes of the mice were determined. The relative index was calculated by means of division of left lymph node indices by similar indices of the right lymph node. Relative indices of weight and cellularity of lymph nodes both for the experimental and control group are equal to 0.95 and 0.98. Thus, evaluating the influence of the herbal extract on the weight and cellularity of lymph nodes, it was established that the preparation does not have allergenic properties.

*3.6.4. Immunotoxic effect*

A study on different immunotoxic properties of the herbal extract was carried out in accordance with WHO recommendations. Examination of the influence of the herbal extract on the humoral immune response was determined by the number of antibody-forming cells in a spleen according to Erne. The influence of the herbal extract on cellular immunity was determined by hypersensitivity reaction of a delayed-type for mice. According to the recommendations of Labor Meeting in Arlington, the influence of the preparation on spleen cellularity against a background of antigenic stimulus was defined.

*3.6.5. Effect on the antount of antibody forming murine spleen cells*

To study the influence of the herbal extract on the amount of antibody-forming cells in spleens of mice, a direct method of local hemolysis was used. It helps to define the cells forming immunoglobuline M-antibody withy a high hemolytic activity. Forty-nine hybrid mice F1 (CBA* C57B16) were selected (males with a body weight equal to 18-20 g). The mice were divided into 7 groups, each group consisted of 7 animals. The mice were immunized with an i.v. injection, of sheep red blood cells (SRBC). On the forth day after immunization the number of antibody forming colonies (AFC) in a mice spleen was defined according to Jerne's method. From acquired results we understood that the herbal extract did not influence the amount of AFC in mice spleens, which were immunized by SRBC using the doses and scheme as mentioned, and correspondingly, did not influence the primary immune response.

*3.6.6. Effect on the number of nucleus-containing murine spleen cells*

Examinations were carried out on 49 mice of F1 (CBA* C57B16; males, the body weight of which was equal to 18-20 g). The animals were divided into 7 groups, every group consisted of 7 mice. They were immunized with an i.v.

injection of SRBC in a dose of $5 \times 10^8$ cells per mouse. The data proved that a single i.m. injection of the herbal extract to mice did not influence the cellularity of the spleen if doses of the preparation equal to 0.18, 0.07 ml/kg were given before the day of immunization, on the day and after the day as well.

3.7. Conclusion

Summing up the result of the toxic study on the herbal extract that was considered to be an immunomodulating drug, we can note that the preparation was clean at a single i.m. injection of the herbal extract to laboratory animals and was well assimilated by Wistar rats and dogs during i.m. injection.

The investigation held showed that a single i.m. injection of the herbal extract diluted 1:10 in normal saline in doses of 0.5-1.0 ml per one mouse (BALB/c mice) did not cause intoxication and death of the animals. An increased dose of the preparation (diluted 1:10 in normal saline in doses of 1.5-2.0 ml per mouse, 75-100 ml/kg) led to a decrease of motor activity and depression in the animals, but their death was not observed.

Intramuscular and i.p. injection of the herbal extract dissolved with 1:5 physiological solution to BALB/c mice was accompanied by a great depression of animals, narcosis and sleep. Animal intoxication with the herbal extract on $LD_{50}$ level was similar to their poisoning by ethyl alcohol, the latter being a part of the preparation.

According to $LD_{50}$ indices, the herbal extract can be classified as belonging to the group of safe preparations if an i.m. injection of 51-66 ml/kg of the substance of the preparation after 1:5 dissolution With a physiological solution occurs. At the same time no significant specific and sexual differences in the sensitivity under conditions of a chronic experiment was observed in Wistar rats treated daily for 3 months by i.m. injection of 0.07 and 0.21 ml/kg of the preparation and in dogs treated daily for 1 month by i.m. injection of 0.07 ml/kg of the herbal extract. The marked doses of the preparation were diluted 1:10 in sterile normal saline before injection. The doses of the herbal extract tested on mice and dogs in chronic experiments exceeded the daily therapeutic dose for humans (0.5 ml/person or 0.007 ml/kg; 10 or 30 times).

Results of the studies showed that doses of 0.07 and 0.21 ml/kg of the herbal extract in a 3 month chronic experiment in mice and 0.07 ml/kg in a 1 month chronic experiment in dogs were well assimilated by animals and did not influence hematologic indices or functional conditions of main organs of the test animals (according to the data of the biochemical tests used and of the ECG). Absence of toxic damages in inner organs, general and local toxico-allergic reactions that are concerned with the effect of the herbal extract was confirmed by the results of pathomorphological investigations, held after the end of chronic experiments. Locally irritating effect of the preparation in chronic experiments in mice and dogs using doses of 0.07 and 0.21 ml/kg at a long-term i.m. injection of 1:10 dilutions in sterile normal saline was not observed. According to requirements of Pharmacological State Committee of Ministry of Public Health, an investigation of mutagenic properties of the herbal extract was held.

Besides, we studied the ability of the preparation to cause gene mutations at indicated cultures of *Salmonella typhimurium* in the Ames test, to stimulate chromosomal aberrations in the cells of hybrid F1(CBA*C57B16) mice bone marrow cells, to influence the amount of dominant lethal mutation in embryonic mice cells and to influence the system of DNA repair of *E. coli* PQ 37 in SOS-chromotest.

During the investigation held it was established that the herbal extract did not have mutagenic properties.

In a dose equal to 0.21 ml/kg (that is 30 times higher than the daily dose recommended for humans), the herbal extract reduced the increase of pregnant rat's body weight upon the i.m. injection for the first to the 20th days of gestation, it also reduced the duration of pregnancy, amount of alive fetuses, places of implantation, yellow bodies and embryo's body weight. At the same time the index of pre-implant death was much lower for the mice that received 0.21 ml/kg of the herbal extract during pregnancy than for control mice, and the indices of post-implant death were lower for the first group.

During a macroscopic examination and microscopic investigation of standard sections of fetuses (according to Wilson-Diban) that underwent the influence of 0.21 ml/kg of the herbal extract during the prenatal period, in 6.7% of all the cases it was possible to suggest about underdevelopment of the fetuses. The effect can be evaluated as a defect of embryo's development.

An analysis of the preparations stained with alizarin that was necessary for studying the development of bone system in rat fetuses exposed to 0.21 ml/kg of the herbal extract during the prenatal period did not show defects of skeleton development. But at the same time a delay of ossification in the majority of points of calcification was observed.

Under the influence of i.m. injection of 0.21 ml/kg of the herbal extract from the first to the 20th days of gestation, a decrease in new born rats and an increase in stillborn rats was noticed as compared with the control group. The body weight of rats exposed to the herbal extract during the prenatal period was lower than the indices of the control group. Results of the experiment on the development of the descendants did not deviate from the time constraints typical for a normal physiological development of this type of animals.

Therefore, the experiments held to establish that an i.m. injection of 0.21 ml/kg of the herbal extract (30-fold the highest daily dose for a human) from the first to the 20th days of gestation has an embryotoxic and teratogenic effect in animals exposed to the herbal extract Therefore, the pregnancy can be considered as a contraindication for prescription of the herbal extract. At a daily i.m. injection of 0.21 ml/kg of the herbal extract to male rats during 10 weeks and female rats during 2 weeks, the influence of the preparation on the reproductive function of animals was not established.

Studying the allergenic properties of the herbal extract on guinea-pigs showed that at 5-fold i.m. injection of the herbal extract in sensitizing doses of 0.07 and 0.14 ml/kg and i.p. injection of a determinant dose of 0.14 ml/kg of the herbal extract at the 14th and 21st days after sensitization, the preparation did not cause anaphylactic shock.

The herbal extract in studied doses and schedules of sensitization did not have an allergenic effect of delayed type hypersensivity reaction in guinea-pigs and in the reaction of the popliteal lymphnode in mice.

In doses of 0.07 ml/kg and 0.18 ml/kg, the herbal extract did not influence the number of antibody-forming and nucleus-containing cells in the spleen, and it did not influences the reaction of hypersensitivity in mice. The data are evidence of the absence of a negative influence of the herbal extract on the humoral and cellular immunity and therefore of the absence of immunotoxicity of the preparation.

Finally, based on all of the experiments conducted and the obtained results, the herbal extract is recommended for clinical trials with the only contraindication of pregnancy.

EXAMPLE 4: Pharmacologic effects of the herbal extract

Studies were conducted in order to determine the herbal extract's potential in patients.

The first study was done in the year 2000. This study was designed for a clinical try on. The first aim of this project was the determination of toxicity or side-effects of the herbal extract in HIV patients, and the next aim was the determination of probable effectiveness of the herbal extract on the course of disease and humoral, cellular and non-specific immunities in the HIV-infected persons.

The 16 to 40 years old HIV positive patients who were at high risk to develop AIDS in general examination were selected for study. The patients used the herbal extract for 80 days. In The 16 to 40 years old HIV positive patients who were at high risk to develop AIDS in general examination were selected for study. The patients used the herbal extract for 80 days. In this project, 0.4 ml of the herbal extract that had been diluted by normal saline up to 4 ml was injected i.m. and i.v. daily. During this period, the patients were examined daily and the effects of the treatment were registered. After a treatment period of 3 months, the patients were pursued and their preclinical factors were studied. The percentages of T-lymphocytes CD4 were determined at $21 \pm 1$ % at the first day of the study, $23 \pm 1.5$ % after 30 days of treat-ment with the herbal extract, $32 \pm 0.8$ % after 60 days of treatment, $32 \pm 0.7$% after 80 days of treatment and $39 \pm 1.6$ % three months after the end of treatment period. These data show an increase in the amount of CD4 T-lymphocytes during treatment with the herbal extract. The percentages of CD8 lymphocytes in patients were $25 \pm 1.5$ % at the first day of the study, $24 \pm 1.5$ % after 30 days of treatment with the herbal extract, $22 \pm 0.8$ % after 60 days of treatment and after 80 days of treatment, it was $23 \pm 0.8$ %. In the follow-up of three months after the end of the treatment it was $20 \pm 2$%.

The percentages of T-lymphocytes including CD95 were $40 \pm 9.2$ % at the first day of the study, $47 \pm 2$ % after 30 days of treatment with the herbal extract, $25 \pm 1.4$ % after 60 days of treatment, $30 \pm 1.3$ % after 80 days of treatment. In the follow-up of three months after the end of the treatment it was $25 \pm 1.5$ %. In the statistical comparison at the first day and 80 days after treatment with the herbal extract, there were an obvious differences between CD4, CD8 and CD95 in the patients ($P<0.01$).

The next study was done for considering the early and late side-effects of the herbal extract in the AIDS patients and HIV infected persons. In this project, six volunteers who were HIV positive were selected. The inclusion criteria were: HIV positive with severe fungi or other opportunistic infection, reduction of body weight more than 10%, secondary zoster, fever for more than one month. After selection of patients and entrance in the project, the preclinical parameters were measured initially and then the measurement was repeated weekly.

0.4 ml of the herbal extract was taken in a 5 ml syringe, diluted with 3.5 ml of warm normal saline and was injected i.m. for two days and than i.v. for two more days. After completion of injections, the patients were examined for the drug side-effects and appearance of AIDS related clinical signs and symptoms. Data are shown in Table 12.

Table 12. Clinical data obtained in the second clinical study on the herbal extract enrolling six volunteers.

| No. | Age | Sex | Weight | CD4 % before | CD4% 3rd month | CD4 count before | CD4 count 3rd month | CD8 % before | CD8% 3rd month | CD8 count before | CD8 count 3rd month |
|---|---|---|---|---|---|---|---|---|---|---|---|
| 1 | 38 | male | 76 | 2 | 12 | 28 | 260 | 62 | 62 | 881 | 1345 |
| 2 | 42 | male | 63 | 21 | 21 | 177 | 318 | 58 | 56 | 490 | 894 |
| 3 | 48 | male | 56 | 22 | 23 | 352 | 636 | 15 | 47 | 688 | 799 |
| 4 | 23 | male | 67 | 22 | 23 | 350 | 360 | 23 | 38 | 318 | 574 |
| 5 | 23 | female | 54 | 24 | 41 | 352 | 651 | 41 | 43 | 632 | 683 |
| 6 | 28 | male | 56 | 19 | 51 | 221 | 1273 | 51 | 25 | 252 | 624 |
| Mean | 33.6 | | 62 | 17.5 | 27.1 | 247 | 517 | 40 | 45 | 543 | 812 |
| P* | | | | 0.04 | | 0.02 | | 0.1 | | 0.02 | |
| *P-value by Wilcoxon signed ranks test | | | | | | | | | | | |

EP 1 978 980 B1

The next study was done for comparing the effectiveness and early side-effects of the herbal extract with routine HAART treatment in recovery of immunological factors in AIDS patients. In this study, 27 volunteers were studied. The patients were randomly divided into two treatment groups treated either with the herbal extract or HARRT (with selection of 4 blocks of randomization blocks). Two patients showed no tolerance in HAART, and thus, they were transferred to the herbal extract group. The patient's entrance and exit criteria were: HIV positive, severe affection by fungi or opportunistic infections now or before, reduction of body weight more than 10%, secondary zoster, fever for more than one month and minor opportunistic affections. These patients had not used any anti-AIDS drugs or enhancement drug for immune system. Antibiotics or other drugs, however, for elimination of AIDS effects was not prohibited. After selection of patients and their entrance into the project, the preclinical factors like blood cell count, triglyceride, cholesterol, uric acid, creatinine, blood urea nitrogen, alkaline phosphatase, aspartate transaminase, alanine transaminase, fasting blood sugar, RNA viral load, CD4 (Thl, Th2), CD4/CD8, CD8, urine analysis/urine culture were measured before treatment and 1, 2, and 3 months after treatment.

In the herbal extract group, 0.4 ml of the herbal extract was taken in a 5 ml syringe and was diluted with 3.5 ml of warm normal saline serum, and the mixture was injected i.m. or i.v. once a day for 90 days. The HAART treatment was done according to the standard regimen. The treatment was 9 tablets of Caplet Nelfmavir 250 mg for 90 days together with 2 capsules of Zidovudine 300 mg for 90 days together with 2 tablets of Lamivudine 150 mg for 90 days. All of the patients were examined for drug's side-effects and appearance of AIDS related clinical signs and symptoms. The data were registered in related questioners. 16 patients in the herbal extract group and 11 patients in the HAART group were examined. The comparisons of characteristics between 2 groups are shown in Table 13.

Table 13. Comparison of the two study groups (WBC - white blood cells).

| Variable | Herbal extract | HAART | P-value |
|---|---|---|---|
| Age (Mean, SD) | 34.9 (8.1) | 38.6 | 0.4 |
| Sex female/male | 4/12 | 0/11 | 0.12 |
| Weight | 62.2 (8) | 57.3 (8) | 0.3 |
| CD4 percent (Mean, SD) | 14.0 (6.0) | 18.3 (13) | 0.5 |
| CD4 number (Mean, SD) | 227 (90) | 239 (136) | 0.8 |
| CD8 percent (Mean, SD) | 39.3 (28.1) | 48.6 (30.1) | 0.6 |
| CD8 number (Mean, SD) | 686 (538) | 624 (408) | 0.8 |
| WBC (Mean, SD) | 5500 (3238) | 4662 (667) | 0.8 |
| Lymphocyte percent (Mean, SD) | 35.5 (12.2) | 31.0 (9.0) | 0.5 |
| Viral load (Mean, SD) | 2161721 (1278272) | 270983 (228802) | 0.4 |

The results in Table 14 indicate that the CD4 number has increased in both groups but there is no significant difference between the groups.

Table 14. Comparison of CD4, CD8, white blood cells (WBC) and lymphocyte percentage in the two study groups.

| Variable | Herbal extract | HAART | P-value |
|---|---|---|---|
| CD4 percent (Mean, SD) | 17.2 (6.6) | 26.2 (8.6) | 0.02 |
| CD4 number (Mean, SD) | 349 (232) | 470 (191) | 0.21 |
| CD8 percent (Mean, SD) | 39.5 (20.5) | 30.4 (19.4) | 0.3 |
| CD8 number (Mean, SD) | 871 (910) | 507 (303) | 0.4 |
| WBC (Mean, SD) | 5242 (1819) | 4900 (2225) | 0.2 |
| Lymphocyte percentage (Mean, SD) | 38.1 (7.7) | 38.2 (5.3) | 0.5 |

Table 15 shows the different parameters in the group of patients who received the herbal extract. The increase in the percentage of CD4 is obvious and evident.

Table 15. Comparison of CD4, CD8, white blood cells and lymphocyte percentage in the herbal extract treated group at the beginning and the end of study

| Variable | Herbal extract (beginning) | Herbal extract (end) | P-value |
|---|---|---|---|
| CD4 percentage (Mean, SD) | 14.0 (6) | 17.2 (6.6) | 0.01 |
| CD4 Number (Mean, SD) | 227 (90) | 349 (232) | 0.002 |
| CD8 percentage (Mean, SD) | 39.3 (28.1) | 39.5 (20.5) | 0.9 |
| CD8 Number (Mean, SD) | 686 (538) | 871 (910) | 0.3 |
| WBC* (Mean, SD) | 5500 (3238) | 5242 (1819) | 0.4 |
| Lymphocyte percentage (Mean, SD) | 35.5 (12.2) | 38.1 (7.7) | 0.06 |
| * WBC - white blood cells | | | |

EXAMPLE 5: Determination of the maximum tolerable dose of the herbal extract

This study was conducted to determine the maximum tolerable dose (MTD) of the herbal extract in HIV infected patients and its possible side-effects and toxicity that can cause dose limitation (dose limiting toxicities, DLTs).

The study protocol was based on the dose escalation method. The effects of the herbal extract on viral load and CD4 count of patients were evaluated as by-products. Four cohorts of patients (3 patients each) were selected and treated for 28 days (4 weeks) with escalated doses of the extract. A base dose of the extract has been determined according to $LD_{10}$ (10% of the lethal dose) in former animal experiments. Patients were observed carefully for signs and symptoms of side-effects and toxicity by physical examination and laboratory workups according to the protocol.

All patients were male in the age of 28-60 years (mean: 41.6 years.). In the first cohort, the daily dose of 2 ml of extract in 100 ml warm normal saline was infused over 0.5-1 hr intravenously for 28 days. No toxicity or major side-effects were observed except for an increase in sweating and weight loss in 2 patients. In the second cohort, three other patients received a daily dose of 4 ml. There were no major side-effects and toxicity in this group. In the third cohort of originally 4 patients, one patient was excluded due to non-compliance and inability for regular daily attendance and the daily dose of 6.7 ml administered. In this group there were not only no major dose limiting toxicity and side-effects but also no minor ones. In the fourth cohort, three other patients received the daily dose of 10 ml, and there were no major side-effects and toxicity in this group too.

In summary, a total of 12 patients was included in the study who were treated for 4 weeks with escalated doses of the herbal extract. There was not toxicity or side-effects in all cohorts.

**Claims**

1. A method for preparing a herbal extract, comprising the following steps:

   (a) providing a plant material derived from *Rosa sp., Urtica dioica* and *Tanacetum vulgare;*
   (b) drying the plant material;
   (c) adding an organic solvent;
   (d) incubating the mixture of plant material and organic solvent;
   (e) obtaining the herbal extract;
   (f1) adding selenium and/or an organic or inorganic salt thereof to a concentration of free selenium in the range of 1 to 100 mg/l; and
   (f2) adding urea
   (g) exposing the herbal extract to a pulsed electromagnetic field, wherein the pulsed electromagnetic field has a frequency in the range of 5 to 750 kHz, a power in the range of 10 to 200 Watt, and a magnetic field strength in the range of 100 to 150 $\mu$Tesla.

2. The method according to claim 1, wherein the electromagnetic field pulse has a sinusoidal, rectangular and/or stochastic shape.

3. The method according to claim 1 or 2, wherein the pulsed electromagnetic field has a frequency in the range of 50 to 350 kHz.

4. The method according to claim 3, wherein the pulsed electromagnetic field has a frequency of about 250 kHz.

5. The method according to any of claims 1 to 4, wherein the pulsed electromagnetic field has a power of 20 to 100 Watt.

6. The method according to claim 5, wherein the pulsed electromagnetic field has a power of about 45 Watt.

7. The method according to any of claims 1 to 6, wherein the exposing in step (g) is carried out for a time period of 2 to 5 minutes.

8. The method according to any of claims 1 to 7, wherein the exposing in step (g) is repeated.

9. The method according to claim 8, wherein the exposing in step (g) is carried out for three times.

10. The method according to any of claims 1 to 9, wherein selenium is added to a concentration of free selenium in the range of 5 to 50 mg/l.

11. The method according to claim 10, wherein the selenium is added to a concentration of free selenium in the range of 10 to 20 mg/l.

12. A use of the herbal extract prepared according to any of claims 1 to 11 for the manufacture of a pharmaceutical composition for the treatment of an HIV infection and/or AIDS in a subject.

13. The use according to claim 12, wherein the subject is a vertebrate.

14. The use according to claim 12 or 13, wherein the subject is not pregnant.


**Patentansprüche**

1. Verfahren zum Herstellen eines Kräuterextrakts, umfassend die folgenden Schritte:

   (a) Bereitstellen eines Pflanzenmaterials gewonnen aus *Rosa sp., Urtica dioica* und *Tanacetuni vulgare;*
   (b) Trocknen des Pflanzenmaterials;
   (c) Zugeben eines organischen Lösungsmittels;
   (d) Inkubieren der Mischung aus Pflanzenmaterial und organischem Lösungsmittel;
   (e) Erhalten des Kräuterextrakts;
   (f1) Zugeben von Selen und/oder eines organischen oder anorganischen Salzes hiervon in einer Konzentration von freiem Selen in dem Bereich von 1 bis 100 mg/l; und
   (f2) Zugeben von Harnstoff;
   (g) Aussetzen des Kräuterextraktes einem gepulsten elektromagnetischen Feld, wobei das gepulsten elektromagnetische Feld eine Frequenz in dem Bereich von 5 bis 750 kHz, eine Leistung in dem Bereich von 10 bis 200 Watt und eine Magnetfeldstärke in dem Bereich von 100 bis 150 μTesla hat.

2. Verfahren nach Anspruch 1, wobei der Puls des elektromagnetischen Feldes eine sinusartige, rechteckige und/oder stochastische Form hat.

3. Verfahren nach Anspruch 1 oder 2, wobei das gepulsten elektromagnetische Feld eine Frequenz in dem Bereich von 50 bis 350 kHz hat.

4. Verfahren nach Anspruch 3, wobei das gepulsten elektromagnetische Feld eine Frequenz von etwa 250 kHz hat.

5. Verfahren nach einem der Ansprüche 1 bis 4, wobei das gepulsten elektromagnetische Feld eine Leistung von 20 bis 100 Watt hat.

6. Verfahren nach Anspruch 5, wobei das gepulsten elektromagnetische Feld eine Leistung von etwa 45 Watt hat.

7. Verfahren nach einem der Ansprüche 1 bis 6, wobei das Aussetzen in Schritt (g) für einen Zeitraum von 2 bis 5 Minuten durchgeführt wird.

8. Verfahren nach einem der Ansprüche 1 bis 7, wobei das Aussetzen in Schritt (g) wiederholt wird.

9. Verfahren nach Anspruch 8, wobei das Aussetzen in Schritt (g) dreimal durchgeführt wird.

10. Verfahren nach einem der Ansprüche 1 bis 9, wobei Selen in einer Konzentration von freiem Selen in dem Bereich von 5 bis 50 mg/l zugegeben wird.

11. Verfahren nach Anspruch 10, wobei das Selen in einer Konzentration von freiem Selen in dem Bereich von 10 bis 20 mg/l zugegeben wird.

12. Verwendung des Kräuterextrakts, hergestellt nach einem der Ansprüche 1 bis 11, zum Herstellen einer pharmazeutischen Zusammensetzung für die Behandlung einer HIV-Infektion und/oder AIDS eines Probanden.

13. Verwendung nach Anspruch 12, wobei der Proband ein Wirbeltier ist.

14. Verwendung nach Anspruch 12 oder 13, wobei der Proband nicht schwanger ist.

**Revendications**

1. Un procédé de préparation d'un extrait de plantes, comprenant les étapes suivantes:

   (a) l'utilisation d'une matière végétale dérivée de *Rosa sp,* d'*Urtica dioica* et de *Tanacetum vulgare;*
   (b) le séchage de la matière végétale;
   (c) l'addition d'un solvant organique;
   (d) l'incubation du mélange de la matière végétale et du solvant organique;
   (e) l'obtention de l'extrait de plantes ;

   (f1) l'addition de sélénium et/ou d'un de ses sels organiques ou inorganiques à une concentration de sélénium libre comprise dans la plage d'environ 1 à 100 mg/l, et
   (f2) l'addition d'urée,

   (g) l'exposition de l'extrait de plantes à un champ électromagnétique pulsé, ce champ électromagnétique pulsé présentant une fréquence comprise dans la plage d'environ 5 à 750 kHz, une puissance comprise dans la plage d'environ 10 à 200 Watt et une intensité du champ magnétique dans la plage de 100 à 150 $\mu$Tesla.

2. Un procédé selon la revendication 1, dans lequel l'impulsion de champ électromagnétique présente une forme sinusoïdale, rectangulaire et/ou stochastique.

3. Un procédé selon la revendication 1 ou 2, dans lequel le champ électromagnétique pulsé présente une fréquence comprise dans la plage de 50 à 350 kHz,

4. Un procédé selon la revendication 3, dans lequel le champ électromagnétique pulsé présente une fréquence d'environ 250 kHz.

5. Un procédé selon l'une quelconque des revendications 1 à 4, dans lequel le champ électromagnétique pulsé présente une puissance de 20 à 100 Watt.

6. Un procédé selon la revendication 5, dans lequel le champ électromagnétique pulsé présente une puissance d'environ 45 Watt.

7. Un procédé selon l'une quelconque des revendications 1 à 6, dans lequel l'exposition dans l'étape (g) est effectuée pendant une période de temps de 2 à 5 minutes.

8. Un procédé selon l'une quelconque des revendications 1 à 6, dans lequel l'exposition dans l'étape (g) est répétée.

9. Un procédé selon la revendication 8, dans lequel l'exposition dans l'étape (g) est réalisée à trois reprises.

10. Un procédé selon l'une quelconque des revendications 1 à 9, dans lequel le sélénium est ajouté à une concentration de sélénium libre dans la plage d'environ 5 à 50 mg/l,

11. Un procédé selon la revendication 10, dans lequel le sélénium est ajouté à une concentration de sélénium libre dans la plage d'environ 10 à 20 mg/l.

12. Une utilisation de l'extrait de plantes selon une quelconque des revendications 1 à 11, pour la fabrication d'une composition pharmaceutique pour le traitement d'une infection par le VIH et/ou le SIDA dans un sujet.

13. L'utilisation selon la revendication 12, dans lequel le sujet est un vertébré.

14. L'utilisation selon la revendication 12 ou 13, dans laquelle le sujet n'est pas enceinte.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- RU 2191824 C2
- RU 2171284 C2

- SU 1169657 A

**Non-patent literature cited in the description**

- Methodical instructions for evaluating allergenic properties of pharmacological substances. Experimental study guide of new pharmacological substances. 2000, 25-32